# FASCICULE DE BREVET EUROPEEN

(11) **EP 1 800 134 B1**
(45) Date de publication et mention de la délivrance du brevet: **26.11.2008**
(21) Numéro de dépôt: 05809109.1
(22) Date de dépôt: 13.10.2005
(51) Int. Cl.: G01N 33/68, G01N 33/567, A61K 39/395, A61K 38/17, C12Q 1/68, A61K 31/70

(54) **PROCEDE DE DIAGNOSTIC ET TRAITEMENT DE LA MALADIE DE CROHN**
VERFAHREN ZUR DIAGNOSE UND BEHANDLUNG VON MORBUS CROHN
METHOD FOR DIAGNOSING AND TREATING CROHN'S DISEASE

(30) Priorité: 14.10.2004 FR 0410865
(43) Date de publication de la demande: 27.06.2007
(73) Titulaire: Université d'Auvergne Clermont 1, 63000 Clermont-Ferrand (FR)
(72) Inventeur: BARNICH, Nicolas, F-63320 Ludesse (FR); DARFEUILLE-MICHAUD, Arlette, 63670 La Roche Blanche (FR); GLASSER, Anne-Lise, F-63170 Aubiere (FR)
(74) Mandataire: Colombet, Alain André
(86) Numéro de dépôt international: PCT/FR2005/002538
(87) Numéro de publication internationale: WO 2006/040481

(56) Documents cités:
- WO-A-97/39356
- WO-A-03/002117
- WO-A-03/070266
- US-A1- 2004 005 304
- US-B1- 6 290 959
- SAUTER SYBILLE L ET AL: "Identification of the specific oligosaccharide sites recognized by type 1 fimbriae from Escherichia coli on nonspecific cross-reacting antigen, a CD66 cluster granulocyte glycoprotein" JOURNAL OF BIOLOGICAL CHEMISTRY, vol. 268, no. 21, 1993, pages 15510-15516, XP002328260 ISSN: 0021-9258 cité dans la demande
- BOUDEAU JEROME ET AL: "Type 1 pili-mediated adherence of Escherichia coli strain LF82 isolated from Crohn's disease is involved in bacterial invasion of intestinal epithelial cells" MOLECULAR MICROBIOLOGY, vol. 39, no. 5, mars 2001 (2001-03), pages 1272-1284, XP002328261 ISSN: 0950-382X cité dans la demande
- DATABASE BIOSIS [Online] BIOSCIENCES INFORMATION SERVICE, PHILADELPHIA, PA, US; avril 2005 (2005-04), BARNICH NICOLAS ET AL: "E. coli colonization of the ileal mucosa in patients with Crohn's disease involves type I pili and expression of the glycosylphosphatidylinositol-anchored molecule CEACAM6" XP002386968 Database accession no. PREV200600211501 & GASTROENTEROLOGY, vol. 128, no. 4, Suppl. 2, avril 2005 (2005-04), page A501, ANNUAL MEETING OF THE AMERICAN-GASTROENTEROLOGICAL-ASSOCIATION/D IGEST IVE-DISEASE-WEEK; CHICAGO, IL, USA; MAY 14 -19, 2005 ISSN: 0016-5085

## Description

L'invention concerne le domaine du diagnostic *in vitro* de la maladie de Crohn. Elle permet à la fois de diagnostiquer la présence de la maladie, et de déterminer la prédisposition d'une personne à développer la maladie. Elle concerne encore le traitement préventif ou curatif de cette maladie.

La maladie de Crohn, décrite en 1932 par Crohn *et al*., est caractérisée par un état d'hyperactivation du système immunitaire de l'intestin, évoluant par poussées entrecoupées de périodes de rémission de durées variables. L'incidence en France, exprimée en nombre de nouveaux cas par an pour 10⁵ habitants, est estimée à 100/10⁵ habitants. En 2004, plus de 100 000 cas ont été recensés en France.

La maladie de Crohn (MC ou CD, pour Crohn's Disease) se manifeste cliniquement par des douleurs abdominales, de la diarrhée, de la fièvre et une dénutrition. Des signes d'inflammation extra-digestive (articulaire, cutanée ou oculaire) sont fréquemment associés. Le diagnostic repose sur l'état clinique, l'endoscopie, les biopsies iléo-coliques et la radiologie de l'intestin grêle. Le tableau clinique, endoscopique et anatomo-pathologique n'a aucune spécificité, et l'établissement du diagnostic nécessite d'exclure toutes les autres affections curables donnant un tableau analogue (Colombel et Menard, 1993). Des tests biologiques sont utilisés pour le diagnostic et le suivi de la maladie de Crohn incluant les marqueurs suivants : ANCA (perinuclear anti-neutrophilic antibody), ASCA (anti-*Saccharomyces* cerevisiae antibody) et anti OmpC (outer membrane porine C from *E*. *coli*). Ces marqueurs ne sont pas spécifiques de la maladie de Crohn et un test négatif ne peut pas éliminer l'éventualité d'une maladie de Crohn (Nakamura et al 2003).

La maladie de Crohn constitue un des problèmes majeurs de l'hépato-gastro-entérologie car elle n'a pas de traitement spécifique. C'est une maladie très invalidante plus ou moins contrôlée par les corticoïdes, les immunosuppresseurs, l'assistance nutritionnelle et les interventions chirurgicales. Le traitement est basé principalement sur des données symptomatiques et n'est que suspensif, car n'aboutissant jamais à la guérison totale du patient. Les principales fonctions de ce traitement sont de contrôler les poussées et ensuite de prévenir la récidive. Schématiquement, les médicaments utilisés se divisent en deux catégories : les anti-inflammatoires tels les dérivés salicylés ou les corticoïdes, et les immunosuppresseurs. Cependant, ces traitements s'avèrent souvent inefficaces et les résections chirurgicales sont inévitables dans 90% des cas. Récemment, l'infliximab, un anticorps monoclonal anti-TNFα, s'est révélé efficace pour le traitement de certains patients présentant un tableau clinique sévère et ne répondant pas aux traitements conventionnels (Rutgeerts et al 1999, Present et al 1999). Toutefois, ce traitement présente des limites. En effet, les patients traités avec l'infliximab produisent parfois des anticorps anti-infliximab, diminuant la durée de réponse au traitement (Baert et al 2003). De plus, certains patients soumis à ce traitement ont développé une pathologie infectieuse grave de type tuberculose, aspergillose, candidose (Wallis et al, 2004).

L'étiologie de la maladie de Crohn n'est pas encore clairement élucidée. Plusieurs facteurs influençant la survenue, le maintien ou l'aggravation de la maladie de Crohn ont été envisagés. Il s'agit en particulier de facteurs environnementaux, immunitaires, génétiques et infectieux (Podolsky 2002, Shanahan 2002).

La maladie de Crohn est caractérisée par un état d'hyperactivation du système immunitaire au niveau intestinal qui se traduit par un recrutement et une activation des lymphocytes et des macrophages. Il est maintenant bien établi que les cytokines pro-inflammatoires telles que interleukine-1 (II-1), II-6, II-8 et TNF-α libérées en grande quantité dans la muqueuse intestinale des patients atteints de maladie de Crohn sont responsables de l'induction de l'inflammation intestinale (Desreumaux et al 1997).

De nombreux arguments cliniques, expérimentaux et épidémiologiques suggèrent l'intervention d'agents infectieux dans la genèse et/ou l'entretien des lésions de maladie de Crohn (Sartor et al 1996). La progression régulière de la maladie, sa répartition en foyers parfois très actifs, et l'apparition de cas familiaux de maladie de Crohn impliquant des couples mariés non apparentés et leurs enfants évoquent le rôle d'un agent infectieux transmissible (Bennett et al 1991).

L'étude de souches de *E*. *coli* isolées de biopsies iléales de patients atteints de maladie de Crohn a montré que les lésions iléales aiguës et chroniques de maladie de Crohn étaient anormalement colonisées par des souches de *E*. *coli* (représentant jusqu'à 100% de la flore totale). Ces souches sont dépourvues des gènes de virulence classiquement retrouvés chez les différents pathovars de *E*. *coli* responsables d'infections intestinales. La majorité (80%) de ces souches possède un fort pouvoir d'adhésion *in vitro* aux cellules épithéliales intestinales Caco-2 et une relation a été mise en évidence entre pouvoir d'adhésion et taux de colonisation de la muqueuse intestinale (Darfeuille-Michaud et al., 1998).

La souche de *E*. *coli* LF82, isolée d'une lésion iléale chronique chez un patient atteint de maladie de Crohn, possède toutes les caractéristiques d'un pathogène bactérien invasif: (i) les bactéries sont internalisées par les cellules épithéliales en culture avec un niveau d'internalisation similaire à ceux de *Shigella,* (ii) son processus d'entrée actif dépend à la fois des microtubules et d'une polymérisation d'actine, et (iii) elle est capable de survivre et de se multiplier dans le cytoplasme de la cellule hôte après lyse de la vacuole d'endocytose.

La caractérisation d'un phénotype d'adhésion-invasion de la souche LF82 et l'absence de déterminants génétiques d'invasion déjà décrits chez *E*. *coli, Shigella* et *Salmonella* a conduit à définir l'existence d'un nouveau groupe pathogène de *E*. *coli* pouvant être associé à la maladie de Crohn, désigné AIEC pour « adherent-invasive *E. coli* » (Boudeau et al., 1999). Ces souches AIEC pourraient coloniser la muqueuse intestinale des malades en adhérant à la bordure en brosse des entérocytes (Darfeuille-Michaud, 2002). Leur prévalence est de 36,4% au niveau des lésions iléales aigües de patients atteints de maladie de Crohn (Darfeuille-Michaud et al 2004).

Une des caractéristiques histologiques de la maladie de Crohn est la présence de granulomes dans 50 à 87% des cas. Une inflammation granulomateuse est retrouvée dans certaines infections bactériennes comme les salmonelloses, yersinioses ou paratuberculoses, mettant en jeu des bactéries invasives possédant également la capacité de résister au pouvoir bactéricide des macrophages. Récemment, il a été montré que chez 80% des patients atteints de MC la présence d'ADN *d'Escherichia coli* dans les granulomes, confirmant la piste infectieuse impliquant *E*. *coli* (Ryan et al. 2004). Après phagocytose par des macrophages murins ou humains *in vitro,* la souche AIEC LF82 survit et se multiplie dans une large vacuole, tout en préservant l'intégrité de la cellule hôte. Suite à l'infection, les macrophages sécrètent un taux important de TNFα. Ainsi, les souches AIEC sont capables non seulement d'envahir les cellules épithéliales mais possèdent également des déterminants génétiques leur permettant de résister à l'action bactéricide des macrophages (Glasser et al., 2001).

Les études de ribotypage n'ont pu mettre en évidence la présence d'une souche unique, par contre des souches AIEC isolées de lésions chroniques ou de récidives présentent un profil de ribotypage identique ou appartiennent à un groupe commun. Ces souches génétiquement liées pourraient avoir évolué à partir d'un même ancêtre par acquisition de facteurs de virulence par transfert horizontal de gènes ou insertion d'îlots de pathogénicité dans le chromosome bactérien (Masseret et al., 2001).

Il a en outre été montré dans un modèle *in vitro* de cellules épithéliales intestinales que les pili de type 1 sont impliqués dans l'adhésion des bactéries AIEC aux cellules et également dans l'internalisation active des bactéries en induisant la formation d'élongations membranaires par la cellule infectée (Boudeau et al., 2001).

Le processus d'adhésion d'une bactérie à des cellules eucaryotes résulte d'une interaction spécifique entre un ligand de la surface bactérienne appelé adhésine et un récepteur cellulaire (Beachey et al 1981). Les récepteurs impliqués dans l'adhésion de souches de *E*. *coli* via les pili de type 1 décrits jusqu'à présents sont des glycoprotéines à pied d'ancrage glycosylphosphatidylinositol (GPI) (Guignot et al., 2000; Leusch et al., 1990; Malaviya et al., 1999; Nowicki et al., 1993). L'adhésine FimH des pili de type 1 reconnaît les récepteurs à pied d'ancrage GPI CD48 et CEACAM6 (CD66c) (Shin et al., 1999 ; Shin et al. ; 2000 ; Sauter et al., 1991 ; Sauter et al., 1993). La glycoprotéine CD66c (CEACAM6, ou encore dénommée NCA pour « Non-specific Cross-reacting Antigen ») appartient à la famille des immunoglobulines, caractérisée par des domaines variables dans la partie N-terminale de la protéine, et des domaines constants contenant des ponts disulfures induisant la formation de boucles (Hammarstrôm et Baranov 2001). CD66c est normalement exprimé à la surface des granulocytes, des macrophages, des polynucléaires neutrophiles, mais également par les cellules épithéliales au niveau du colon, du poumon et de la rate (Audette et al., 1987; Kolbinger et al., 1989; von Kleist et al., 1972; Jantscheff et al., 2003).

WO-A-03/002117 concerne un complexe chimique comportant un ou plusieurs dérivés carboxy de pyridines et un ou plusieurs aminosucres. Il est précisé que l'aminosucre peut être un dérivé de monosaccharide ou de di-saccharide et que dans le cas où il s'agit d'un mono-saccharide, il est choisi dans un groupe de plusieurs composés incluant le mannosamine et celui-ci peut être en outre choisi parmi des sulfates, un hydrochloride ou encore N-acétylmannosamine.
Ce complexe est utilisable pour la préparation d'un produit destiné à la suppression de l'hypersensibilité et/ou à la suppression de réactions inflammatoires chez un mammifère. Cette utilisation peut viser le traitement de maladies dermatologiques, de maladies rhumatismales, de problèmes articulaires, du traitement d'allergie ou encore du traitement de maladies auto-immunes ou de maladies inflammatoires chroniques. Un grand nombre de maladies sont visées potentiellement par l'invention et parmi celles-ci se trouve citée la maladie de Crohn. Les exemples exposés dans ce document concernent des applications topiques ou dermatologiques.

US-A-2004/005304 divulgue une composition pour le traitement de désordres neurologiques et de maladies intestinales associées. Cette composition comprend un principe actif qui peut être choisi parmi différentes classes de composes, à savoir peptide, saccharide, probiotique et métal ainsi que des combinaisons de ceux-ci. Parmi les saccharides, ce document indique qu'il peut s'agir de mono ou de poly-saccharide et parmi les mono-saccharides, le mannose est mentionné. Par ailleurs, les maladies visées sont classées en deux groupes, d'une part les maladies neurologiques, d'autre part les maladies gastro-intestinales et parmi cette dernière catégorie se trouvent citées la maladie de Crohn ainsi qu'un grand nombre d'autres maladies.

WO-A-97/39356 décrit une méthode de diagnostic de la maladie de Crohn basée sur la détection d'anticorps pANCA et propose de la compléter par la détection d'anticorps anti-Saccharomyces cerevisiae qui reconnaissent des épitopes à motif mannose.

En raison de la gravité de l'affection, et des difficultés actuelles concernant son diagnostic, un diagnostic précoce et fiable de prédisposition ou d'atteinte de maladie de Crohn est évidemment très hautement souhaitable. Il contribuerait à éviter les temps d'attente importants, toujours pénalisants pour les patients, qui séparent actuellement la prise en charge du patient de l'établissement du diagnostic après exclusion de toutes les autres affections donnant un tableau clinique analogue.

Enfin, il existe aussi un besoin, d'une méthode permettant de prévenir la maladie de Crohn et/ou de traiter cette maladie, qui soit mieux ciblée que les méthodes actuellement employées.

Les auteurs de la présente invention se sont donc attachés à mettre au point des méthodes alternatives pour le diagnostic et la thérapie de la maladie de Crohn. Au coeur de l'invention se trouve la mise en évidence, faite par les inventeurs, d'une surexpression anormale du récepteur CD66c au niveau iléal chez les patients atteints de la maladie de Crohn et d'une affinité remarquable entre ce récepteur et les souches d'*E. coli* AIEC. Les données obtenues par les inventeurs montrent, d'une part, que le récepteur est surexprimé au niveau de la bordure en brosse des entérocytes et, d'autre part, que les souches *E. coli* de type AIEC ont une affinité très élevée pour ces récepteurs, ce qui expliquerait la colonisation de la muqueuse iléale par les bactéries AIEC. L'adhésion des souches AIEC au récepteur CD66c s'effectue par l'intermédiaire des pili de type 1. La souche de référence de ce groupe AIEC, à savoir la souche LF82, exprime des pili de type 1 variants présentant plusieurs substitutions d'acides aminés dans la sous-unité majeure FimA, dans les sous-unités mineures Fiml et FimF et dans l'adhésine FimH (J. Boudeau et al., 2001). Les auteurs de la présente invention ont en outre constaté que 51 % des souches d'*E. coli* isolées de patients atteints de la maladie de Crohn présentaient des pili de type 1 variants, ayant des substitutions d'acides aminés dans FimH similaires à celles observées pour FimH de la souche de référence LF82. Ces pili de type 1 variants pourraient donc être responsables de la plus grande affinité de ces souches pour le récepteur CD66c.

### Applications diagnostiques

La présente invention a donc pour objet un procédé *in vitro* de diagnostic de la maladie de Crohn, ou de détermination d'une prédisposition d'une personne à développer la maladie de Crohn, dans lequel on détermine si le niveau d'expression du récepteur CD66c dans un échantillon biologique d'un sujet à tester est supérieur au niveau d'expression d'un échantillon contrôle, ce qui est indicatif de la maladie de Crohn, ou d'un prédisposition du sujet testé à développer la maladie de Crohn.

Dans le contexte de l'invention, un « échantillon biologique » peut être une biopsie iléale ou une préparation d'entérocytes isolés à partir d'une biopsie iléale. Par « biopsie iléale », on entend un prélèvement d'une partie de l'iléon ou de la muqueuse iléale, par exemple obtenu lors d'une résection chirurgicale (on parle alors de pièce opératoire, prélevée au niveau de l'iléon) ou lors d'une endoscopie. L'échantillon biologique peut également être un échantillon d'un fluide biologique, tel que du sang ou du sérum, ou encore d'un échantillon de selles.

Lorsque le procédé de l'invention est mis en oeuvre sur un échantillon biologique autre qu'une biopsie iléale ou une préparation d'entérocytes isolés - à savoir notamment quand l'échantillon biologique est un échantillon de sang ou de selles-, il est avantageux d'associer à la détection de CD66c, une détection (quantitative ou semi-quantitative) de marqueurs tumoraux tels que le CEA (« carcinonoembryonic antigen ») (Grunert et al., 1995). Ainsi, on peut discriminer directement entre patients atteints de cancers colorectaux et patients atteints de la maladie de Crohn.

Le procédé de l'invention implique soit la détermination quantitative du niveau d'expression absolu du récepteur CD66c, puis la comparaison avec le niveau d'expression du récepteur chez un sujet contrôle, déterminé en parallèle ou connu par ailleurs, soit la détermination directe du niveau d'expression relatif du récepteur CD66c dans l'échantillon biologique à tester par rapport à l'échantillon contrôle (on peut alors parler de détection semi-quantitative). L'échantillon « contrôle » est un échantillon d'un sujet « sain » ou un sujet non atteint de maladie de Crohn, ou un sujet non atteint de toute maladie inflammatoire de l'intestin (IBD) ou de cancer colorectal. Il peut, selon le cas, s'agir d'un sujet présentant des lésions inflammatoires de l'intestin grêle d'origine traumatique ou infectieuse. Afin de déterminer l'évolution de la maladie de Crohn, il peut être utile de déterminer chez un sujet le niveau d'expression de CD66c, et de contrôler l'effet d'un médicament ou le développement de la maladie, en testant le sujet une seconde fois, par exemple plusieurs semaines plus tard. Dans ce cas, les résultats du second test sont comparés avec les résultats du premier test et en général également avec les résultats obtenus chez un sujet « sain ». L'échantillon « contrôle » se réfère ainsi soit au même sujet test soit au sujet « sain ».

Un « sujet » ou « patient » est un mammifère, de préférence un humain, quels que soient son sexe, son âge et sa condition générale. Les enfants sont également compris. Le sujet testé peut être asymptomatique, ou peut être considéré comme susceptible de développer la maladie de Crohn.

Le terme « diagnostic » se réfère à la détermination ou à la confirmation d'une atteinte par la maladie de Crohn, quel que soit son stade de développement. Il peut s'agir plus particulièrement d'un diagnostic précoce ou d'un diagnostic de récidive.

Le niveau d'expression du récepteur CD66c peut être déterminé de différentes manières. Il peut être notamment déterminé en dosant le récepteur CD66c ou en déterminant son niveau de transcription, c'est-à-dire la quantité d'ARNm qui code pour le récepteur. Il peut également être déterminé par la détection et/ou la quantification des motifs mannose, dont le CD66c est riche. Différentes méthodes pour la détection et/ou la quantification de l'expression du récepteur CD66c sont décrites plus bas.

Selon un premier mode de réalisation, le niveau d'expression du récepteur CD66c est déterminé par la mesure de la quantité de protéine récepteur CD66c, généralement en mettant en contact un échantillon biologique avec un partenaire de liaison capable d'interagir de manière sélective avec le récepteur CD66c présent dans l'échantillon. Le partenaire de liaison est généralement un anticorps, qui peut être polyclonal ou monoclonal, de préférence monoclonal. Il peut également s'agir d'un fragment peptidique du récepteur CD66c, tel qu'un des peptides décrits dans la demande de brevet WO01/013937, et reportés dans le tableau 1 ci-après :

**Tableau 1 :**

| Nom du peptide | Séquence peptidique |
|---|---|
| CD66c-1 | STPFNVAEGKEVL |
| CD66c-2 | LAHNLPQNRIGYSW |
| CD66c-3 | KGERVDGNSLIVGY |
| CD66c-4 | VGYVIGTQQATPG |
| CD66c-5 | ATPGPAYSGRETIY |
| CD66c-6 | LLIQNVTQNDTGFY |
| CD66c-7 | VIKSDLVNEEATGQ |
| CD66c-8 | EATGQFHVYPELPK |
| CD66c-9 | NNSNPVEDKDAVAF |
| CD66c-10 | PEVQNTTYLWWVNG |
| CD66c-11 | NGQSLPVSPRLQLS |
| CD66c-12 | LQLSNGNMTLTLLS |
| CD66c-13 | TLLSVKRNDAGSYE |

### Anticorps

Le terme *« anticorps »* se réfère à tout anticorps entier ou fragment fonctionnel d'un anticorps (obtenu par génie génétique ou non), comprenant, ou consistant en, au moins un site de combinaison antigénique, permettant audit anticorps de se lier à au moins un déterminant antigénique d'un composé antigénique. A titre d'exemple de fragments d'anticorps on peut citer les fragments Fab, Fab', F(ab')₂ ainsi que les fragments variables à simple chaîne (chaînes scFv).

Par « anticorps de capture », on entend un anticorps ou une partie d'anticorps, de préférence fixé sur une phase solide, qui est capable de retenir l'antigène CD66c présent dans un échantillon biologique, par liaison affine.

La présence de l'antigène dans l'échantillon biologique est révélée par des « moyens de détection ». S'agissant de la détection de l'antigène, l'invention prévoit notamment une détection à l'aide d'au moins un « anticorps de détection ». Un tel anticorps de détection, marqué, est capable de se lier à l'antigène capturé, par liaison affine, en reconnaissant un site épitopique, différent de celui reconnu par l'anticorps de capture.

Le terme « marqué » se réfère aussi bien à un marquage direct (par le biais d'enzymes, radioisotopes, fluorochromes, composés luminescents, etc) qu'à un marquage indirect (par exemple, par le biais d'anticorps eux-mêmes marqués de manière directe ou à l'aide de réactifs d'une « paire d'affinité » marquée, telle que, mais non exclusivement, la paire avidine marquée-biotine, etc..).

Par « fragment antigénique », on entend toute partie du CD66c capable d'induire la synthèse d'anticorps substantiellement spécifique du seul CD66c chez un animal immunisé.

Le terme « spécifiquement », quand il se réfère à une reconnaissance ou une liaison spécifique d'un anticorps pour un antigène, signifie que l'anticorps interagit avec l'antigène sans interaction substantielle avec d'autres antigènes.

Les anticorps anti-CD66c utilisés dans la présente invention sont des anticorps spécifiques de l'antigène. Ce sont de préférence des anticorps monoclonaux ou des anticorps polyclonaux monospécifiques, c'est à dire qu'ils ne reconnaissent spécifiquement qu'un épitope.

La production d'anticorps monoclonaux ou de sérums polyclonaux monospécifiques, ou d'anticorps obtenus par criblage de banques génomiques, utiles dans le cadre de l'invention relève de techniques conventionnelles.

Un anticorps polyclonal anti-CD66c peut, entre autres, être obtenu par immunisation d'un animal tel qu'un lapin, une souris, etc.. à l'aide du récepteur CD66c soluble ou d'un fragment antigénique de celui-ci, prélèvement puis épuisement de l'antisérum obtenu sur, par exemple, un immunoadsorbant contenant le récepteur selon des méthodes en soi connues de l'homme du métier.

Plusieurs anticorps monoclonaux ont été développés et commercialisés. On peut ainsi utiliser l'anticorps monoclonal anti-CD66c clone 9A6 (Genovac, Suisse) ou l'anticorps monoclonal anti-CD66c d'lnnoGenex, Menarini Diagnostics, (Anthony, France).

De manière générale, d'autres anticorps monoclonaux peuvent être obtenus selon la méthode classique de fusion lymphocytaire et culture d'hybridomes décrite par Köhler et Milstein, (1975). D'autres méthodes de préparation d'anticorps monoclonaux sont également connues (Harlow et al, ed., 1988 « Antibodies : a laboratory manual »). Les anticorps monoclonaux peuvent être préparés en immunisant un mammifère (par exemple une souris, un rat, un lapin, voire un être humain, etc...) et en utilisant la technique de fusion lymphocytaire conduisant à des hybridomes (Köhler et Milstein, 1975).

Des techniques alternatives à cette technique usuelle existent. On peut, par exemple, produire des anticorps monoclonaux par expression d'un acide nucléique cloné à partir d'un hybridome. On peut également produire des anticorps par la technique d'expression sur phage (« phage display »), en introduisant des ADNc d'anticorps dans des vecteurs, qui sont typiquement des phages filamenteux qui présentent des banques de gènes V à la surface du phage (par exemple fUSE5 pour *E.coli,* Scott, 1990). Des protocoles de construction de ces banques d'anticorps sont décrits dans Marks et al, (1991).

Les trousses et réactifs utiles pour la détection du CD66c dans un échantillon biologique, conformément au procédé de l'invention, peuvent être fournis pour une mise en pratique de l'invention facile et applicable à de nombreux échantillons biologiques.

Des trousses de détection du CD66c dans un échantillon biologique peuvent contenir au moins un anticorps tel que défini précédemment.

### Tests immunologiques

La quantité de glycoprotéine récepteur CD66c-est ainsi de préférence mesurée par un test immunologique, comprenant la mise en contact de l'échantillon biologique avec un anticorps anti-CD66c, éventuellement marqué, qui reconnaît spécifiquement CD66c, et la révélation des complexes anticorps-récepteurs CD66c formés.

Par exemple, la présence de CD66c peut être détectée en utilisant des techniques standards d'électrophorèse et d'immunodiagnostic, incluant des immunoessais, par exemple par compétition, réaction directe ou de type sandwich. De tels essais incluent notamment des Western Blots, des tests d'agglutination, des immunoessais par marquage enzymatique (ELISA), des essais de type avidine/biotine, des radioimmunoessais, des immunoélectrophorèses, des immunoprécipitations, etc. Les réactions incluent généralement la révélation de marqueurs tels que des molécules fluorescentes, chimioluminescentes, radioactives ou des marqueurs enzymatiques, ou encore des colorants.

Le procédé de diagnostic selon l'invention peut être réalisé selon divers formats bien connus de l'homme du métier : en phase solide ou en phase homogène ; en un temps ou en deux temps ; en méthode sandwich ou en méthode compétitive, à titre d'exemples non limitatifs.

Selon un mode de réalisation préféré, l'anticorps de capture est immobilisé sur une phase solide. On peut utiliser, à titre d'exemples non limitatifs de phase solide, des microplaques, en particulier des microplaques de polystyrène, telles que celles commercialisées par la société Nunc, Danemark. On peut également utiliser des particules ou des billes solides, des billes paramagnétiques, telles que celles fournies par Dynal ou Merck-Eurolab (France) (sous la marque Estapor^{™}), ou encore des tubes à essai en polystyrène ou polypropylène, etc...

Un format d'immunoessai de type sandwich entre deux anticorps (de capture et de détection) est particulièrement avantageux pour la détection des antigènes présents dans l'échantillon biologique, lorsque celui-ci est un fluide biologique tel que du sang, ou un échantillon de selles par exemple.

Un format d'immunoessai de détection des antigènes par compétition est également possible. D'autres modalités d'immunoessai sont encore envisageables et bien connues de l'homme du métier.

Dosages ELISA, radioimmunoessais, ou toute autre technique de détection peuvent être mis en oeuvre pour révéler la présence des complexes antigènes-anticorps formés.

Selon un mode de réalisation préféré, un procédé *in vitro* de diagnostic de la maladie de Crohn comprend un test immunologique, par exemple de type test ELISA, sur un échantillon de sang ou de selles. On peut ainsi mettre en oeuvre un dosage immunologique sur plaque, avec un anticorps de capture CD66abce, fixé et différentes dilutions de sang ou de selles, l'antigène CD66c étant révélé par un anticorps de révélation spécifique, à savoir un anticorps anti-CD66c marqué, par exemple couplé à une enzyme ou un fluorochrome. L'anticorps anti-CD66c peut être par exemple l'anticorps monoclonal de souris anti-CD66abce humain de DAKO (clone Kat4C, DAKO, Danemark). On peut également utiliser de la Concanavaline A à la place de l'anticorps fixé, pour capturer le récepteur anti-CD66c par ses motifs mannose.

De manière alternative, le niveau d'expression du récepteur CD66c est déterminé par la mesure du niveau de production d'anticorps anti-CD66c. Le niveau d'expression d'anticorps anti-CD66c peut être déterminé, de préférence dans un échantillon de sang ou de selles, par la mise en contact de l'échantillon, avec un antigène CD66c, éventuellement fixé, ou un fragment épitopique de celui-ci, et la révélation des complexes anticorps- récepteurs CD66c formés. On peut ainsi mettre en oeuvre un dosage immunologique sur plaque, avec l'antigène CD66c fixé et différentes dilutions de sang ou de selles, les anticorps anti-CD66c étant révélés par un anticorps anti-IgG, ou anti-IgA, ou encore anti-IgM marqué, par exemple couplé à une enzyme ou un fluorochrome.

Selon un autre mode de réalisation, le procédé de l'invention comprend un test d'immunohistochimie réalisé sur une biopsie iléale.

Par exemple, des coupes de biopsie iléale fixées peuvent être marquées par un anticorps anti-CD66c, puis révélées avec un anticorps secondaire couplé à un fluorochrome ou à une enzyme de type peroxydase ou phosphatase alcaline. L'expression du récepteur CD66c peut être interprétée, après observation au microscope ou lecture électronique, et classée selon un score anatomo-pathologique compris entre 0 (absence d'expression) et 4 (très forte expression au niveau cytoplasmique et bordure en brosse), indiquant le stade d'avancement de la maladie.

Alternativement, des tests de marquage peuvent être réalisés en mettant en présence des préparations d'entérocytes isolés à partir de biopsie iléale, avec un anticorps anti-CD66c directement marqué de manière détectable, par exemple par un fluorochrome de type FITC ou Alexa Fluor 488 ou 647. On visualise ainsi le marquage spécifique des récepteurs CD66c surexprimés au niveau de la bordure en brosse des entérocytes dans le cas de maladie de Crohn.

### Détection des motifs mannose

Selon un autre mode de réalisation de l'invention, le niveau d'expression du récepteur CD66c est déterminé de manière indirecte par la détection quantitative des motifs mannose présents au niveau de la bordure en brosse des entérocytes. Ce type de test est particulièrement pratique à mettre en oeuvre sur des biopsies iléales ou des préparations d'entérocytes isolés.

Il permet de mettre facilement en évidence la surexpression des récepteurs CD66c, qui sont fortement mannosylés, au niveau de la bordure en brosse des entérocytes. Par exemple la détection quantitative ou semi-quantitative des motifs mannose peut être réalisée par la mise en contact de l'échantillon biologique avec une lectine telle que la Concanavaline A marquée de manière détectable, et la révélation des complexes formés par la Concanavaline A liée aux motifs mannose. La concanavaline A peut être par exemple couplée à un fluorochrome, tel que le FITC.

### Tests ARN

Selon un autre mode de réalisation de l'invention, le niveau d'expression du récepteur CD66c est déterminé par la mesure de la quantité d'ARNm de CD66c dans l'échantillon biologique.

Les procédés pour déterminer le niveau de transcription d'un gène sont bien connus. Par exemple, l'acide nucléique contenu dans les échantillons (par exemple une préparation d'entérocytes ou une biopsie iléale), est généralement d'abord extrait selon les méthodes standards, par exemple à l'aide d'enzymes lytiques, ou de solutions chimiques par des résines liant les acides nucléiques. L'ARNm extrait est ensuite détecté par hybridation (par exemple par analyse Northern Blot) et/ou amplication après transcription inverse (RT-PCR), à l'aide d'amorces oligonucléotidiques spécifiques. Un exemple d'amorces spécifiques pouvant être utilisées est donné ci-après :
CD66c F : 5' CCTGCAGATTGCATGTCCCC (SEQ ID N° 1)
CD66c R : 5' CCAATACGATTCTGGGGCAGG (SEQ ID N° 2)

Dans un exemple particulier de réalisation, l'échantillon biologique est une biopsie iléale ou une préparation d'entérocytes isolés et la quantité d'ARNm de CD66c est mesurée par RT-PCR en temps réel, de préférence après extraction des ARN totaux.

### Applications thérapeutiques

Un autre aspect de l'invention fournit des méthodes thérapeutiques pour la prévention ou le traitement de la maladie de Crohn. Ces méthodes sont basées sur la mise en évidence que le récepteur CD66c est surexprimé chez les patients atteints de la maladie de Crohn et est responsable de la liaison des bactéries *Escherichia coli* AIEC à la bordure en brosse des entérocytes. Tout agent inhibant spécifiquement l'interaction entre le récepteur CD66c et les souches d'*E. coli* AIEC est donc utile pour la prévention ou le traitement de la maladie de Crohn.

L'invention vise donc également l'utilisation d'un agent inhibant spécifiquement l'interaction entre le récepteur CD66c et les souches d'*Escherichia coli* AIEC pour la fabrication d'un médicament destiné à la prévention ou au traitement de la maladie de Crohn.

Cet agent peut être un anticorps anti-CD66c inhibiteur, qui reconnaît spécifiquement le récepteur CD66c et bloque la liaison de ce récepteur avec une souche d'*E. coli* AIEC.

Ledit agent peut être de manière alternative une glycoprotéine récepteur CD66c ou une glycoprotéine de synthèse mimant CD66c.

Il peut également s'agir d'un fragment peptidique du récepteur CD66c, tel qu'un des peptides décrits dans la demande de brevet WO01/013937, et reportés ci-dessus.

L'agent peut également être un mannoside, tel que du D-mannose, du méthyl-D-mannose, ou une particule portant un ou des motifs mannoses. Par définition, le terme mannoside inclut donc le D-mannose et les composés susceptibles de libérer du D-mannose par hydrolyse, par exemple les polyholosides et les oligoholosides libérant du D-mannose par hydrolyse (homo- ou hétéroholosides), ainsi que tous dérivés du D-mannose susceptibles d'interagir avec l'adhésine FimH des souches AIEC. Les particules portant un ou des motifs mannoses peuvent être par exemple des billes ou particules inertes ou des cellules vivantes ou tuées.

Plus particulièrement, l'agent utilisé peut être un agent qui bloque spécifiquement l'interaction entre le récepteur CD66c et l'adhésine FimH des souches AIEC.

L'invention fournit par ailleurs un procédé *in vitro* de criblage de substances candidates pour la prévention ou le traitement de la maladie de Crohn, comprenant :
(i) la mise en contact du récepteur CD66c exprimé à la surface d'une cellule, avec au moins une souche d'*Escherichia coli* AIEC, en présence ou en absence d'une substance à tester ;
(ii) la détermination de la capacité de la substance à inhiber spécifiquement l'interaction entre le récepteur CD66c et ladite souche, et la sélection et/ou l'identification de ladite substance.

Des substances candidates peuvent être de n'importe quel type, incluant des composés naturels ou synthétiques ou des mélanges de composés. La substance peut être structurellement définie ou d'une structure inconnue, par exemple sous la forme d'un extrait biologique.

Pour déterminer la capacité de la substance candidate à inhiber la liaison entre les souches *E. coli* AIEC et le récepteur CD66c, des tests de compétition standards peuvent être mis en oeuvre sur des cultures cellulaires exprimant le récepteur CD66c. Il peut s'agir par exemple de cellules transformées génétiquement pour surexprimer le récepteur, ou d'entérocytes isolés de biopsie iléale de patients atteints de la maladie de Crohn. Il peut s'agir de cellules épithéliales intestinales cultivées en monocouches (à titre d'exemples les cellules HT29, Caco-2, T84, Intestine-407). Dans le cas où la substance candidate est un composé identifié, il peut être marqué, par exemple par un marqueur radioactif ou non radioactif, (par exemple fluorescéine).

Le marqueur spécifiquement lié au récepteur CD66c peut ensuite être quantifié en présence d'une concentration variable de ladite substance candidate, par exemple de 10⁻¹⁰ à 10⁻⁵ M. De manière alternative, on peut suivre la compétition de la substance candidate avec la bactérie *E*. *coli* AIEC vis-à-vis du récepteur CD66c par des tests d'adhésion.

Les agents qui inhibent spécifiquement l'interaction entre le récepteur CD66c et les souches d'*E*. *coli* AIEC peuvent être formulés sous la forme de composition pharmaceutique, de préférence pour une administration par voie orale.

Ces compositions peuvent être administrées par voie orale par exemple, par exemple sous forme de comprimés, de gélules ou de granules à libération immédiate ou contrôlée.

Une composition solide pour une administration orale est préparée par addition au principe actif d'une charge et, le cas échant, d'un liant, d'un agent délitant, d'un lubrifiant, d'un colorant, ou d'un correcteur de goût, et par mise en forme du mélange en un comprimé, un comprimé enrobé, un granulé, une poudre ou une capsule.

Des exemples de charges englobent le lactose, l'amidon de maïs, le saccharose, le glucose, le sorbitol, la cellulose cristalline et le dioxyde de silicium, et des exemples de liants englobent le poly(alcool vinylique), le poly(éther vinylique), l'éthylcellulose, la méthylcellulose, l'acacia, la gomme adragante, la gélatine, le Shellac, l'hydroxypropylcellulose, l'hydroxypropylméthylcellulose, le citrate de calcium, la dextrine et la pectine.

Des exemples de lubrifiants englobent le stéarate de magnésium, le talc, le polyéthylène glycol, la silice, et les huiles végétales durcies. Le colorant peut être n'importe lequel de ceux autorisés pour une utilisation dans les médicaments.

Des exemples de correcteurs de goût comprennent le cacao en poudre, la menthe sous forme d'herbe, la poudre aromatique, la menthe sous forme d'huile, le bornéol et la cannelle en poudre. Il doit être compris que le comprimé ou le granulé peut être convenablement enrobé de sucre, de gélatine ou analogue.

Les doses et posologies efficaces d'administration des agents thérapeutiques, destinées à la prévention ou au traitement de la maladie de Crohn, dépend d'un grand nombre de facteurs, et par exemple de la nature de l'agent, de la taille du patient, du stade de la maladie, de la composition pharmaceutique spécifique utilisée et des observations et des conclusions du médecin traitant.

Par exemple, dans le cas d'une administration par voie orale, par exemple un comprimé ou une gélule, une posologie possible convenable se situe entre environ 0,1 mg/kg et environ 100 mg/kg de poids corporel par jour, de préférence entre environ 0,5 mg/kg et environ 50 mg/kg de poids corporel par jour, plus préférentiellement entre environ 1 mg/kg et environ 10 mg/kg de poids corporel par jour et de préférence encore entre environ 2 mg/kg et environ 5 mg/kg de poids corporel par jour de matière active.

Si l'on considère des poids corporels représentatifs de 10 kg et 100 kg afin d'illustrer la gamme de posologie journalière par voie orale qui peut être utilisée et comme décrit ci-dessus, des posologies convenables, seront comprises entre environ 1-10 mg et 1000-10 000 mg par jour, de préférence entre environ 5-50 mg et 500-5000 mg par jour, de préférence encore entre environ 10,0-100,0 mg et 100,0-1000,0 mg par jour, et plus préférentiellement entre environ 20,0-200,0 mg et environ 50,0-500,0 mg par jour, du principe actif.

Ces gammes de posologie représentent des quantités totales de principe actif par jour pour un patient donné. Le nombre d'administration par jour auquel une dose est administrée peut varier dans de grandes proportions, notamment en fonction des facteurs pharmacocinétiques.

Les figures et exemples suivants illustrent l'invention sans en limiter la portée.

### Légende des figures :

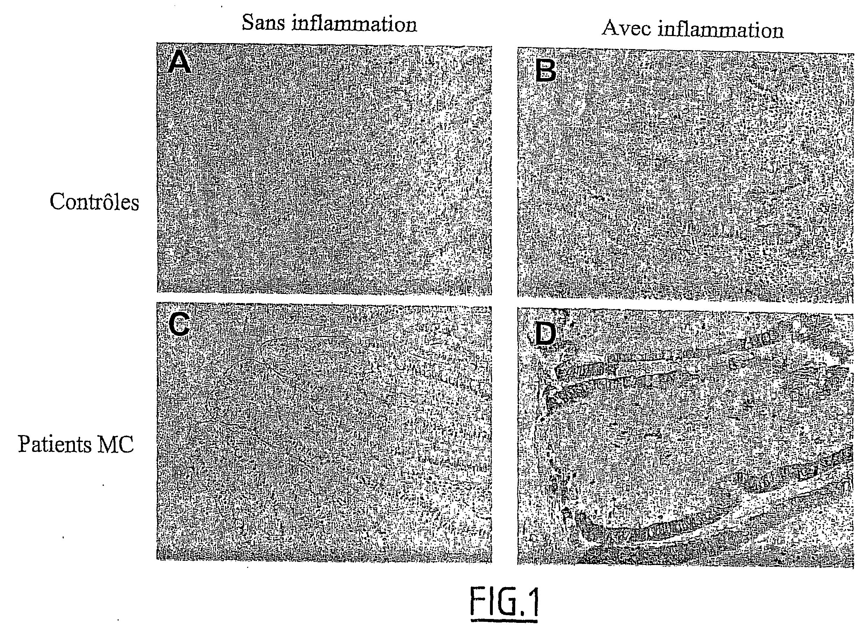

La figure 1 représente des coupes de muqueuse iléale chez des sujets non atteints de maladie inflammatoire de l'intestin de type maladie de Crohn ou rectocolite hémorragique (1A et 1B) et chez des sujets atteints de la maladie de Crohn (1C et 1D). Les figures 1A et 1C sont des coupes de sections de muqueuse sans inflammation, et les figures 1B et 1D sont des coupes de sections de muqueuse avec inflammation comme en témoigne la présence de polynucléaires neutrophiles infiltrés. La figure 1A ne montre aucun marquage avec l'anticorps anti CD66c indiquant que cette glycoprotéine n'est pas exprimée dans le tissu sain. Il est à noter que même dans le tissu présentant des signes d'inflammation (figure 1 B) d'un sujet non atteint de la maladie de Crohn dont l'inflammation intestinale est d'origine traumatique (perforation du grêle sur bride), aucun marquage avec l'anticorps CD66c n'est observé. Ainsi la présence de la glycoprotéine CD66c au niveau iléal n'est pas liée au statut inflammatoire de la muqueuse. La figure 1C met en évidence une expression de CD66c au niveau de la bordure en brosse des entérocytes d'un patient atteint de la maladie de Crohn. Cette expression est observée en absence d'inflammation au niveau d'une muqueuse non ulcérée. La figure 1D présente une hyperexpression de CD66c au niveau de la bordure en brosse des entérocytes ainsi qu'une expression cytoplasmique de cette protéine en zone inflammatoire et lésée chez un patient atteint de la maladie de Crohn. L'immunomarquage est réalisé à l'aide d'anticorps anti-CD66c marqués au complexe peroxydase avidine-biotine, couplé à un chromogène.

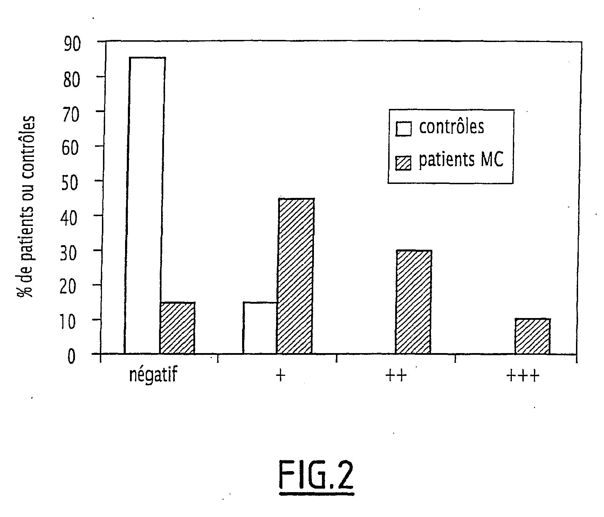

La figure 2 est un graphe représentant le pourcentage de patients ou sujets contrôles montrant un marquage par anticorps anti-CD66c (selon le protocole de la figure 1). Le marquage est noté de - à +++ selon l'intensité. Il apparaît que 85% des patients atteints de la maladie de Crohn expriment de façon anormale le récepteur CD66c au niveau iléal contre seulement 15% des sujets sains.

### EXEMPLES:

### Exemple 1 : Identification de CD66c comme récepteur spécifique des bactéries AIEC impliqué dans la maladie de Crohn :

### A. Souches bactériennes et culture

La souche *E*. *coli* LF82, isolée d'une lésion iléale chronique d'un patient atteint de la maladie de Crohn, est utilisée comme souche de référence (Adherent-Invasive *E*. *coli*). Cette souche est décrite dans J. Boudeau et al. (1999). Elle adhère et envahit les cellules épithéliales intestinales en culture et se réplique dans le cytoplasme des cellules hôtes après la lyse des vacuoles d'endocytose. Elle est capable de survivre et de se répliquer dans les macrophages murins J774-A1 en induisant une forte sécrétion de cytokine pro-inflammatoire TNF-alpha. Elle se multiplie également dans des macrophages péritonéaux de souris et dans des macrophages humains dérivés de monocytes.

On a aussi utilisé ici des mutants de la souche LF82 n'exprimant pas de pili de type 1 suite à une insertion du transposon Tn5*phoA* dans le gène *fimA* (mutant 52D11) ou dans le gène *fimH* (mutant ZG2) de l'opéron fim codant pour les pili de type 1, tels que décrits dans J. Boudeau et al. (2001).

Les bactéries sont cultivées dans du bouillon Luria-Bertani (LB) ou sur des plaques d'agar LB ou Mueller-Hinton (MH) (Institut Pasteur Production, Marnes-la-Coquette, France).

### B. Echantillons iléaux de patients atteints de MC et de témoins sains

Des entérocytes ont été isolés de pièces opératoires iléales obtenus par chirurgie ou par biopsies endoscopiques iléales de 12 patients atteints de la maladie de Crohn (patients CD ou MC) et de 7 sujets ne présentant pas de maladie inflammatoire de l'intestin (« inflammatory bowel disease » IBD). Les échantillons ont été congelés à -80 °C dans du milieu de Eagle modifié (MEM, Seromed, Biochrom, Berlin, Allemagne) contenant 10 % de glycérol et 10 % de diméthylsulfoxyde (DMSO, Sigma Chemical Company, St-Louis, Mo.) immédiatement après prélèvement.

### C. Adhésion in vitro aux entérocytes isolés

Les essais d'adhésion ont été conduits comme décrits dans Darfeuille-Michaud et al., (1990) ; S. Knutton et al., (1985) et B. Lafeuille et al., (1981). Les échantillons intestinaux congelés ont été lavés 3 fois dans du PBS et la muqueuse iléale a été raclée avec une lamelle pour détacher les entérocytes en tampon PBS additionné de 0,25 M d'EDTA . Environ 10⁵ entérocytes isolés ont été mélangés à 10⁸ cellules *E*. *coli* dans un milieu MEM contenant 10% de sérum de veau feotal (FCS, Seromed). Après 2 heures d'incubation, à 37 °C et sous agitation modérée, les entérocytes ont été lavés 3 fois dans du PBS (pH 7,2). L'adhésion des bactéries a été quantifiée en examinant au microscope à contraste de phase à un grossissement x1000. Les comptages ont été réalisés en double aveugle pour déterminer le nombre d'*E*. *coli* adhérant à la bordure en brosse de 30 à 50 entérocytes. L'indice d'adhésion est exprimé en nombre de bactéries attachées à la bordure en brosse d'un entérocyte.

Dans le cas des entérocytes provenant de malades, l'indice d'adhésion constaté allait de 0,542 à 1,999 contre 0,096 à 0,511 pour les témoins (tableau 2).

**Tableau 2 : indice d'adhésion des bactéries aux entérocytes**

| | Indice d'adhésion^{a} | |
|---|---|---|
| Patients CD | Moyenne | Ecart-type |
| Patient 1 | 1,999 | 0,459 |
| Patient 2 | 1,429 | 0,106 |
| Patient 3 | 1,352 | 0,101 |
| Patient 4 | 1,599 | 0,294 |
| Patient 5 | 1,459 | 0,196 |
| Patient 6 | 1,426 | 0,417 |
| Patient 7 | 0,768 | 0,167 |
| Patient 8 | 0,542 | 0,090 |
| Patient 9 | 0,551 | 0,083 |
| Patient 10 | 1,404 | 0,092 |
| Patient 11 | 1,056 | 0,347 |
| Patient 12 | 0,708 | 0,337 |
| Patient 13 | 1,017 | 0,117 |
| | | |

| Sujets sains | | |
|---|---|---|
| Sujet 1 | 0,208 | 0,084 |
| Sujet 2 | 0,096 | 0,021 |
| Sujet 3 | 0,133 | 0,030 |
| Sujet 4 | 0,380 | 0,020 |
| Sujet 5 | 0,240 | 0,030 |
| Sujet 6 | 0,222 | 0,028 |
| Sujet 7 | 0,511 | 0,035 |

| | | |
|---|---|---|
| ^{a} nombre moyen de bactéries adhérant à la bordure en brosse d'un entérocyte. | | |

Un indice d'adhésion supérieur à 1 est considéré comme indicatif d'une adhésion efficace à la bordure en brosse (S. Knutton et al., (1985) ; B. Larfeuille et al., (1981)). Dans l'expérience, on a constaté des indices supérieurs à 1 avec les entérocytes de 9 patients CD sur 13, soit 69%, et aucun indice supérieur à 1 pour les témoins. La capacité de la souche LF82 à adhérer à la bordure en brosse des entérocytes est significativement plus élevée avec les entérocytes isolés de patients CD qu'avec les témoins (*P*=0,006). Ceci a conduit à considérer que les patients CD pourraient exprimer un récepteur situé sur la bordure en brosse des entérocytes iléaux, récepteur qui serait impliqué dans l'adhésion de la souche AIEC LF82.

### D. Analyse de la présence de résidus mannose libres sur la bordure en brosse des entérocytes

L'adhésine FimH, située sur les pili de type 1, agit comme une lectine et reconnaît le motif mannosyl des glycolipides ou des glycoprotéines exprimées à la surface des cellules hôtes. Sur cette base, on a utilisé la concanavaline A (ConA) qui lie spécifiquement les résidus mannose pour rechercher la présence de résidus mannose libres sur la bordure en brosse des entérocytes des patients CD et des témoins. Les entérocytes isolés ont été incubés 1 heure en PBS contenant 50 µg/ml de ConA -FITC (ConA marquée à la FITC, Sigma). Après 4 lavages, les entérocytes ont été observés au microscope à fluorescence au grossissement x400.

L'observation a montré que la lectine se liait à la bordure en brosse des entérocytes des patients CD. En revanche, aucune fixation de la lectine aux entérocytes témoins n'a été observée, ce qui démontre l'expression d'une molécule fortement mannosylée sur la bordure en brosse des entérocytes de patients CD, qui n'est pas observée chez les témoins.

### E. Expression de CD66c dans des échantillons iléaux obtenus par résection chirurgicale

Des échantillons de muqueuse inflammatoire et de muqueuse saine ont été obtenus par résection chirurgicale sur 20 patients CD, respectivement sur 20 témoins. A partir de fragments fixés à l'AFA (alcool-formol-acide acétique) puis inclus en paraffine et préparation de coupes minces de 4 µm., on a réalisé des immunomarquages à l'aide d'un appareil automatique d'immunomarquage par anticorps anti-CD66c (Ventana Medical System), en utilisant la méthode au complexe peroxydase avidine-biotine. Les coupes ont été soumises à un traitement à la chaleur de 3 minutes sous pression à la cocotte-minute pour découvrir les épitopes.

Comme anticorps, on a utilisé l'anticorps monoclonal anti-CD66c clone 9A6 (Genovac, Suisse) à la dilution 1 :50.

Les coupes ont été colorées par l'hématoxyline de Mayer.

Les résultats sont présentés aux figures 1 et 2. 85 % des sujets sains n'ont aucun marquage par cet antigène CD66c. En revanche, les patients atteints de la maladie de Crohn montrent une surexpression des récepteurs de CD66c au niveau de la bordure en brosse des cellules épithéliales. Cette expression est très accrue dans le cas de muqueuse présentant une forte inflammation et des ulcérations.

### Exemple 2 : Tests d'inhibition de l'adhésion des bactéries

### A. Essais d'inhibition par le D-mannose

On a testé le pouvoir inhibiteur du D-mannose sur l'adhésion de la souche *E*. *coli* LF82 à la bordure en brosse des entérocytes via les pili de type 1. Des essais d'adhésion ont été conduits en présence de 2 % (p/v) de D-mannose (Sigma) dans du MEM contenant 10 % de FCS, et en procédant comme décrit ci-dessus sur des entérocytes provenant de 3 patients CD (A, B et C). Les résultats sont présentés au tableau 3.

**Tableau 3 :**

| Patients MC | Indice d'adhésion sans D-mannose | Indice d'adhésion (% relatif)^{a} avec 2% de D-mannose |
|---|---|---|
| Patient A | 1,010 | 0,028 (2,7 %) |
| Patient B | 1,511 | 0.032 (2,1 %) |
| Patient C | 1,800 | 0,025 (1,4 %) |

| | | |
|---|---|---|
| ^{a} Pourcentage du pouvoir d'adhésion de la souche LF82 de type sauvage en présence de 2 % de D-mannose, par rapport à celui en l'absence de D-mannose, défini comme représentant 100 %. | | |

L'addition de D-mannose fait significativement diminuer l'indice d'adhésion de la souche LF82 à la bordure en brosse des entérocytes de patients atteints de la maladie de Crohn.

On a aussi testé des mutants de la souche LF82, à savoir le mutant 52D11 n'exprimant pas de pili de type 1 et le mutant ZG2 n'exprimant pas l'adhésine FimH. Ces mutants ont perdu leur capacité d'adhésion à la bordure en brosse, comme l'attestent les résultats présentés dans le tableau 4.

**Tableau 4 :**

| Souches bactériennes | Gène inactivé | Indice d'adhésion^{a} (% relatif)^{b} sur entérocytes du patient D | Indice d'adhésion^{a} (% relatif)^{b} sur entérocytes du patient E |
|---|---|---|---|
| Mutant 52D11 | *fimA* | 0,100(7,0) | 0,150 (10,6) |
| Mutant ZG2 | *fimH* | 0,095 (6,7) | 0,050 (3,6) |
| | | | |
| Souche sauvage LF82 | | 1,426 (100) | 1,404 (100) |

| | | | |
|---|---|---|---|
| ^{a} Nombre moyen de bactéries adhérant à la bordure en brosse d'un entérocyte. ^{b} Pourcentage du pouvoir d'adhésion du mutant par rapport à celui de la souche LF82 de type sauvage, défini comme représentant 100 %. | | | |

Ces résultats confirment que les pili de type 1, et plus particulièrement l'adhésine FimH de la souche LF82 sont impliqués dans l'adhésion à la bordure en brosse des entérocytes iléaux de patients atteints de la maladie de Crohn.

### B. Essai d'inhibition par anticorps

Anticorps monoclonaux testés : monoclonal anti-CD66c (InnoGenex, Menarini Diagnostics, Anthony, France) et monoclonal anti-CD48 (PeliCluster, Tebu, Le Perray en Yvelines, France). Ces anticorps sont utilisés à la dilution 1/100 dans du MEM contenant 10 % de FCS.

Les entérocytes sont prétraités 30 min à 37 °C avec les anticorps. Les bactéries LF82 sont ensuite ajoutées et les essais d'adhésion conduits comme décrit *supra.*

Les résultats sont présentés dans le tableau 5 :

**Tableau 5 :**

| Patients CD | Adhésion aux entérocytes en présence de (% relatif) | | |
|---|---|---|---|
| | Aucun anticorps | Anti-CD48 | Anti-CD66c |
| Patient F | 1,450 ± 0,212 | 1,378 ± 0,174 (95,0) | 0,200 ± 0,067 (13,8) |
| Patient G | 1,145 ± 0,115 | 0,850 ± 0,050 (74,2) | 0,100 ± 0,033 (8,7) |
| Patient H | 1,016 ± 0,116 | 1,027 ± 0,027 (101,1) | 0,267 ± 0,067 (26,3) |
| Patient I | 1,283 ± 0,117 | 1,516 ± 0,051 (118,2) | 0,283 ± 0,050 (22,0) |

| | | | |
|---|---|---|---|
| ^{a} Pourcentage d'adhésion de la souche LF82 à des bordures en brosse d'entérocytes après préincubation avec les différents anticorps, par rapport celui obtenu avec des entérocytes en absence d'anticorps, défini comme représentant 100 %. | | | |

Le prétraitement avec l'anticorps anti-CD66c fait diminuer drastiquement l'adhésion de la souche LF82. En revanche l'anticorps anti-CD48 n'affecte pas l'adhésion, indiquant une spécificité de l'inhibition observée avec l'anticorps CD66c. Ces résultats confirment que l'adhésion des la souche LF82 à la muqueuse iléale de patients CD implique une liaison des pili de type 1 aux récepteurs CD66c surexprimés au niveau de la bordure en brosse des cellules épithéliales intestinales.

### BIBLIOGRAPHIE

Audette, M., F. Buchegger, M. Schreyer, and J. P. Mach 1987. Monoclonal antibody against carcinoembryonic antigen (CEA) identifies two new forms of crossreacting antigens of molecular weight 90,000 and 160,000 in normal granulocytes Mol Immunol. 24:1177-86.
Baert, F., Noman, M., Vermeire, S., Van Assche, G., G, D.H., Carbonez, A. and Rutgeerts, P. (2003) Influence of immunogenicity on the long-term efficacy of infliximab in Crohn's disease. N Engl J Med. 348: 601-608.
Beachey, E.H. (1981) Bacterial adherence: adhesin-receptor interactions mediating the attachment of bacteria to mucosal surface. J Infect Dis. 143: 325-345.
Bennett, R.A., Rubin, P.H. and Present, D.H. (1991) Frequency of inflammatory bowel disease in offspring of couples both presenting with inflammatory bowel disease. Gastroenterology. 100: 1638-1643.
Boudeau, J., A. L. Glasser, E. Masseret, B. Joly, and A. Darfeuille-Michaud 1999. Invasive ability of an Escherichia coli strain isolated from the ileal mucosa of a patient with Crohn's disease Infect Immun. 67:4499-509.
Boudeau, J., N. Barnich, and A. Darfeuille-Michaud 2001. Type 1 pili-mediated adherence of Escherichia coli strain LF82 isolated from Crohn's disease is involved in bacterial invasion of intestinal epithelial cells Mol Microbiol. 39:1272-84.
Colombel, J.F. and Mesnard, B. (1993) Maladie de Crohn. In : Editions Techniques, Encycl. Med.Chir. (Paris, France). Gastroentérologie,. 9-057-G10,: 1-15.
Darfeuille-Michaud, A., D. Aubel, G. Chauviere, C. Rich, M. Bourges, A. Servin, and B. Joly 1990. Adhesion of enterotoxigenic Escherichia coli to the human colon carcinoma cell line Caco-2 in culture Infect Immun. 58:893-902.
Darfeuille-Michaud A. Adherent-invasive Escherichia coli: a putative new E. coli pathotype associated with Crohn's disease. Int J Med Microbiol 2002;292:185 93.
Darfeuille-Michaud, A., Neut, C., Barnich, N., Lederman, E., Di Martino, P., Desreumaux, P., et al (1998) Presence of adherent Escherichia coli strains in ileal mucosa of patients with Crohn's disease. Gastroenterology. 115: 1405-1413.
Darfeuille-Michaud, A., Boudeau, J., Bulois, P., Neut, C., Glasser, AL., Barnich, N., Bringer, MA., Swidsinski, A., Beaugerie, L., Colombel, JF. High prevalence of adherent-invasive Escherichia coli associated with ileal mucosa in Crohn's disease. Gastroenterology 2004, 127, 412-421.
Desreumaux, P., Brandt, E., Gambiez, L., Emilie, D., Geboes, K., Klein, O., et al (1997) Distinct cytokine patterns in early and chronic ileal lesions of Crohn's disease. Gastroenterology. 113: 118-126.
Glasser, A. L., J. Boudeau, N. Barnich, M. H. Perruchot, J. F. Colombel, and A. Darfeuille-Michaud 2001. Adherent Invasive Escherichia coli Strains from Patients with Crohn's Disease Survive and Replicate within Macrophages without Inducing. Host Cell Death Infect Immun. 69:5529-37.
Grunert, F., S. Daniel, G. Nagel, S. von Kleist, and P. Jantscheff 1995. CD66b, CD66c and carcinoembryonic antigen (CEA) are independently regulated markers in sera of tumor patients. Int J Cancer. 63:349-55.
Guignot, J., I. Peiffer, M. F. Bernet-Camard, D. M. Lublin, C. Carnoy, S. L. Moseley, and A. L. Servin 2000. Recruitment of CD55 and CD66e brush border-associated glycosylphosphatidylinositol-anchored proteins by members of the Afa/Dr diffusely adhering family of Escherichia coli that infect the human polarized intestinal Caco-2/TC7 cells Infect Immun. 68:3554-63.
Hammarstom, S., and V. Baranov. 2001. Is there a role for CEA in innate immunity in the colon ? Trends in Microbiol. 9, 119-125.
Jantscheff P, Terracciano L, Lowy A, Glatz-Krieger K, Grunert F, Micheel B, Brummer J, Laffer U, Metzger U, Herrmann R, Rochlitz C. 2003. Expression of CEACAM6 in resectable colorectal cancer: a factor of independent prognostic significance. J Clin Oncol. 21:3638-46.
Köhler et Milstein, (1975), Nature (London), 256 : 495-497
Kolbinger, F., K. Schwarz, F. Brombacher, S. von Kleist, and F. Grunert 1989. Expression of an NCA cDNA in NIH/3T3 cells yields a 110K glycoprotein, which is anchored into the membrane via glycosyl-phosphatidylinositol Biochem Biophys Res Commun. 161:1126-34.
Knutton, S., D. R. Lloyd, D. C. Candy, and A. S. McNeish 1985. Adhesion of enterotoxigenic Escherichia coli to human small intestinal enterocytes Infect Immun. 48:824-31.
Lafeuille, B., A. Darfeuille, S. Petit, B. Joly, and R. Cluzel 1981. ["In vitro" study of attachment to human enterocytes in "Escherichia coli" strains isolated from infants with diarrheal disease (author's transl)] Ann Microbiol (Paris). 132B:57-6
Leusch, H. G., S. A. Hefta, Z. Drzeniek, K. Hummel, Z. Markos-Pusztai, and C. Wagener 1990. Escherichia coli of human origin binds to carcinoembryonic antigen (CEA) and non-specific crossreacting antigen (NCA) FEBS Lett. 261:405-9.
Malaviya, R., Z. Gao, K. Thankavel, P. A. van der Merwe, and S. N. Abraham 1999. The mast cell tumor necrosis factor alpha response to FimH-expressing Escherichia coli is mediated by the glycosylphosphatidylinositol- anchored molecule CD48 Proc Natl Acad Sci U S A. 96:8110-5.
Marks JD, Hoogenboom HR, Bonnert TP, McCafferty J, Griffiths AD, Winter G. 1991. By-passing immunization. Human antibodies from V-gene libraries displayed on phage.J. Mol. Biol. 222, 581-597.
Masseret, E., Boudeau, J., Colombel, J.F., Neut, C., Desreumaux, P., Joly, B., et al (2001) Genetically related Escherichia coli strains associated with Crohn's disease. Gut. 48: 320-325.
Nakamura RM., Matsutani M., Barry M 2003. Advances in clinical laboratory tests for inflammatory bowel disease. Clinic Chimica Acta 335:9-20.
Nowicki, B., Hart, A., Coyne, K.E., Lublin, D.M. and Nowicki, S., Short consensus repeat-3 domain of recombinant decay-accelerating factor is recognized by Escherichia coli recombinant Dr adhesin in a model of a cell-cell interaction. J Exp Med, 178, 2115-21. (1993).
Podolsky DK. Inflammatory bowel disease. N Engl J Med 2002;347:417-29.
Present, D.H., Rutgeerts, P., Targan, S., Hanauer, S.B., Mayer, L., van Hogezand, R.A., et al (1999) Infliximab for the treatment of fistulas in patients with Crohn's disease. N Engl J Med. 340: 1398-1405.
Rutgeerts, P., D'Haens, G., Targan, S., Vasiliauskas, E., Hanauer, S.B., Present, D.H., et al (1999) Efficacy and safety of retreatment with anti-tumor necrosis factor antibody (infliximab) to maintain remission in Crohn's disease. Gastroenterology. 117: 761-769.
Ryan P., Kelly RG., Lee G., Collins JK, O'Sullivan GC., O'Connell JO., Shanahan F. 2004. Bacterial DNA within granulomas of patients with Crohn's disease-Detection by laser capture microdissection. Am J Gastroenterology, 99: 1539-43.
Sartor RB, H. C. Rath, R. K. Sellon. Microbial factors in chronic intestinal inflammation. Current opinion in gastroenterology 1996;12:327-333.
Sauter, S. L., S. M. Rutherfurd, C. Wagener, J. E. Shively, and S. A. Hefta 1991. Binding of nonspecific cross-reacting antigen, a granulocyte membrane glycoprotein, to Escherichia coli expressing type 1 fimbriae Infect Immun. 59:2485-93.
Sauter , S. L., S. M. Rutherfurd, C. Wagener, J.E. Shively, and S.A. Hefta Identification of the specific oligosaccharide sites recognized by type 1 fimbriae from E. coli on non-specific cross-reacting antigen, a CD66 cluster granulocyte glycoprotein. J Biol Chem. 268(21):15510-6
Scott, J.K. and Smith, G.P., 1990, Searching for Peptide Ligands with an Epitope Library. Science, 249 :386-390
Shanahan F. Crohn's disease. Lancet 2002;359:62-9.
Shin,J. S., and S. N. Abraham 1999, Bacteria-host cell interaction mediated by cellular cholesterol/glycolipid-enriched microdomains. Biosci Rep. 19(5):421-32
Shin, J. S., Z. Gao, and S. N. Abraham 2000. Involvement of cellular caveolae in bacterial entry into mast cells Science. 289:785-8.
von Kleist, S., G. Chavanel, and P. Burtin 1972. Identification of an antigen from normal human tissue that crossreacts with the carcinoembryonic antigen. Proc Natl Acad Sci U S A. 69:2492-4.
Wallis RS.; Broder MS., Wong JY., Hanson ME, Beenhouver DO 2004. Granulomatis infectious diseases associated with tumor necrosis factor antagonists. Clin Infect Dis. 38:1261-5.
WO-A-03/002117
US-A-2004/005304
WO-A-97/39356

## Revendications

1. Procédé *in vitro* de diagnostic de la maladie de Crohn, ou de détermination d'une prédisposition d'une personne à développer la maladie de Crohn, dans lequel on détermine si le niveau d'expression du récepteur CD66c dans un échantillon biologique d'un sujet à tester, est supérieur au niveau d'expression dans un échantillon contrôle, ce qui est indicatif de la maladie de Crohn, ou d'une prédisposition du sujet testé à développer la maladie de Crohn.

2. Procédé selon la revendication 1, dans lequel l'échantillon biologique est une biopsie iléale ou une préparation d'entérocytes isolés à partir d'une biopsie iléale.

3. Procédé selon la revendication 1, dans lequel l'échantillon biologique est un échantillon de sang ou de selles.

4. Procédé selon l'une des revendications 1 à 3, dans lequel le niveau d'expression du récepteur CD66c est déterminé par la mesure de la quantité de glycoprotéine récepteur CD66c.

5. Procédé selon la revendication 4, dans lequel la quantité de protéine récepteur CD66c est mesurée par un test immunologique, comprenant la mise en contact de l'échantillon biologique avec un anticorps anti-CD66c, éventuellement marqué, qui reconnaît spécifiquement CD66c, et la révélation des complexes anticorps-récepteurs CD66c formés.

6. Procédé selon la revendication 5, dans lequel l'anticorps anti-CD66c est un anticorps monoclonal.

7. Procédé selon la revendication 6, dans lequel l'échantillon biologique est une biopsie iléale, et le test immunologique est un test d'immunohistochimie.

8. Procédé selon la revendication 6, dans lequel l'échantillon biologique est un échantillon de sang ou de selles, et le test immunologique est un test ELISA.

9. Procédé selon la revendication 2, dans lequel le niveau d'expression du récepteur CD66c est déterminé de manière indirecte par la détection quantitative des motifs mannose présents au niveau de la bordure en brosse des entérocytes.

10. Procédé selon la revendication 9, dans lequel la détection quantitative des motifs mannose est réalisée par la mise en contact de l'échantillon biologique avec la Concanavaline A marquée de manière détectable, et la révélation des complexes formés par la Concanavaline A liée aux motifs mannose.

11. Procédé selon l'une des revendications 1 à 3, dans lequel le niveau d'expression du récepteur CD66c est déterminé par la mesure de la quantité d'ARNm de CD66c dans l'échantillon biologique.

12. Procédé selon la revendication 11, dans lequel l'échantillon biologique est une biopsie iléale et la quantité d'ARNm de CD66c est mesurée par RT-PCR en temps réel.

13. Procédé selon la revendication 3, dans lequel le niveau d'expression du récepteur CD66c est déterminé par la mesure du niveau de production d'anticorps anti-CD66c.

14. Procédé selon la revendication 13, comprenant la mise en contact d'un échantillon de sang ou de selles à tester, avec un antigène CD66c, éventuellement fixé, ou un fragment épitopique de celui-ci, et la révélation des complexes anticorps- récepteurs CD66c formés.

15. Utilisation d'un anticorps anti-CD66c, qui reconnaît spécifiquement le récepteur CD66c, pour la fabrication d'un médicament destiné à la prévention ou au traitement de la maladie de Crohn.

16. Utilisation d'une protéine récepteur CD66c ou une glycoprotéine mimant CD66c, pour la fabrication d'un médicament destiné à bloquer spécifiquement l'interaction entre le récepteur CD66c et les souches d'*Escherichia Coli* AIEC, pour la prévention ou le traitement de la maladie de Crohn.

17. Utilisation d'un mannoside ou d'une particule portant un ou des motifs mannose pour la fabrication d'un médicament destiné à bloquer spécifiquement l'interaction entre le récepteur CD66c et les souches d'*Escherichia Coli* AIEC, pour la prévention ou le traitement de la maladie de Crohn.

18. Procédé *in vitro* de criblage de substances candidates pour la prévention ou le traitement de la maladie de Crohn, comprenant :
(i) la mise en contact du récepteur CD66c, sous forme soluble ou exprimé à la surface d'une cellule, avec au moins une souche d'*Escherichia coli* AIEC, en présence ou en absence d'une substance à tester ;
(ii) la détermination de la capacité de la substance à inhiber spécifiquement l'interaction entre le récepteur CD66c et ladite souche, et la sélection et/ou l'identification de ladite substance.

19. Anticorps anti-CD66c, qui reconnaît spécifiquement le récepteur CD66c, pour utilisation comme médicament pour la prévention ou le traitement de la maladie de Crohn.

20. Médicament comprenant un anticorps anti-CD66c qui reconnaît spécifiquement le récepteur CD66c, pour la prévention ou le traitement de la maladie de Crohn.

21. Protéine récepteur CD66c ou glycoprotéine mimant CD66c pour utilisation comme médicament destiné à bloquer spécifiquement l'interaction entre le récepteur CD66c et les souches d'*Escherichia coli* AIEC pour la prévention ou le traitement de la maladie de Crohn.

22. Médicament comprenant une protéine récepteur CD66c ou une glycoprotéine mimant CD66c, destiné à bloquer spécifiquement l'interaction entre le récepteur CD66c et les souches d'*Escherichia coli* AIEC, pour la prévention ou le traitement de la maladie de Crohn.

23. Mannoside ou particule portant un ou des motifs mannose pour utilisation comme médicament destiné à bloquer spécifiquement l'interaction entre le récepteur CD66c et les souches d'*Escherichia coli* AIEC, pour la prévention ou le traitement de la maladie de Crohn.

24. Médicament comprenant un mannoside ou une particule portant un ou des motifs mannose destiné à bloquer spécifiquement l'interaction entre le récepteur CD66c et les souches d'*Escherichia coli* AIEC, pour la prévention ou le traitement de la maladie de Crohn.

## Claims

1. *In vitro* method for diagnosing Crohn's disease or for determining a predisposition of a person to develop Crohn's disease, in which it is determined whether the level of expression of the CD66c receptor in a biological sample from a test subject is greater than the level of expression in a control sample, which is indicative of Crohn's disease or of a predisposition of the tested subject to develop Crohn's disease.

2. Method according to claim 1, in which the biological sample is an ileal biopsy or a preparation of enterocytes isolated from an ileal biopsy.

3. Method according to claim 1, in which the biological sample is a sample of blood or of stools.

4. Method according to any one of claims 1 to 3, in which the level of expression of the CD66c receptor is determined by measuring the quantity of CD66c receptor glycoprotein.

5. Method according to claim 4, in which the quantity of CD66c receptor protein is measured by an immunological test which comprises bringing the biological sample into contact with an optionally labelled anti-CD66c antibody which specifically recognises CD66c and revealing the antibody-CD66c receptor complexes that are formed.

6. Method according to claim 5, in which the anti-CD66c antibody is a monoclonal antibody.

7. Method according to claim 6, in which the biological sample is an ileal biopsy and the immunological test is an immunohistochemical test.

8. Method according to claim 6, in which the biological sample is a sample of blood or of stools and the immunological test is an ELISA test.

9. Method according to claim 2, in which the level of expression of the CD66c receptor is determined indirectly by the quantitative detection of the mannose units present in the brush border of the enterocytes.

10. Method according to claim 9, in which the quantitative detection of the mannose units is carried out by bringing the biological sample into contact with detectably labelled Concanavalin A and revealing the complexes formed by Concanavalin A bound to the mannose units.

11. Method according to any one of claims 1 to 3, in which the level of expression of the CD66c receptor is determined by measuring the quantity of CD66c mRNA in the biological sample.

12. Method according to claim 11, in which the biological sample is an ileal biopsy and the quantity of CD66c mRNA is measured by real-time RT-PCR.

13. Method according to claim 3, in which the level of expression of the CD66c receptor is determined by measuring the level of production of anti-CD66c antibodies.

14. Method according to claim 13, which comprises bringing a sample of blood or of stools to be tested into contact with an optionally fixed CD66c antigen, or with an epitope fragment thereof, and revealing the antibody-CD66c receptor complexes that are formed.

15. Use of an anti-CD66c antibody which specifically recognises the CD66c receptor in the production of a medicament for the prevention or treatment of Crohn's disease.

16. Use of a CD66c receptor protein or of a glycoprotein that mimics CD66c in the production of a medicament for specifically blocking the interaction between the CD66c receptor and AIEC *Escherichia coli* strains, for the prevention or treatment of Crohn's disease.

17. Use of a mannoside or of a particle carrying one or more mannose units in the production of a medicament for specifically blocking the interaction between the CD66c receptor and AIEC *Escherichia coli* strains, for the prevention or treatment of Crohn's disease.

18. *In vitro* method for screening candidate substances for the prevention or treatment of Crohn's disease, comprising:
(i) bringing the CD66c receptor, in soluble form or in a form expressed at the surface of a cell, into contact with at least one AIEC *Escherichia coli* strain, in the presence or absence of a test substance;
(ii) determining the ability of the substance to specifically inhibit the interaction between the CD66c receptor and said strain, and selecting and/or identifying said substance.

19. Anti-CD66c antibody which specifically recognises the CD66c receptor, for use as a medicament for the prevention or treatment of Crohn's disease.

20. Medicament comprising an anti-CD66c antibody which specifically recognises the CD66c receptor, for the prevention or treatment of Crohn's disease.

21. CD66c receptor protein or glycoprotein that mimics CD66c, for use as a medicament for specifically blocking the interaction between the CD66c receptor and AIEC *Escherichia coli* strains, for the prevention or treatment of Crohn's disease.

22. Medicament comprising a CD66c receptor protein or a glycoprotein that mimics CD66c, for specifically blocking the interaction between the CD66c receptor and AIEC *Escherichia coli* strains, for the prevention or treatment of Crohn's disease.

23. Mannoside or particle carrying one or more mannose units for use as a medicament for specifically blocking the interaction between the CD66c receptor and AIEC *Escherichia coli* strains, for the prevention or treatment of Crohn's disease.

24. Medicament comprising a mannoside or a particle carrying one or more mannose units, for specifically blocking the interaction between the CD66c receptor and AIEC *Escherichia coli* strains, for the prevention or treatment of Crohn's disease.

## Patentansprüche

1. *In vitro*-Verfahren zur Diagnose des Morbus Crohn oder zur Bestimmung einer Veranlagung einer Person, an Morbus Crohn zu erkranken, wobei bestimmt wird, ob das Expressionsniveau des CD66c-Rezeptors in einer biologischen Probe einer zu testenden Person höher ist als das Expressionsniveau in einer Kontrollprobe, was auf Morbus Crohn oder eine Veranlagung der getesteten Person, an Morbus Crohn zu erkranken, hinweist.

2. Verfahren nach Anspruch 1, wobei die biologische Probe eine Ileumbiopsie oder ein Präparat aus Enterozyten ist, die aus einer Ileumbiopsie isoliert wurden.

3. Verfahren nach Anspruch 1, wobei die biologische Probe eine Blut- oder Stuhlprobe ist.

4. Verfahren nach einem der Ansprüche 1 bis 3, wobei das Expressionsniveau des CD66c-Rezeptors durch die Messung der Menge des CD66c-Rezeptor-Glycoproteins bestimmt wird.

5. Verfahren nach Anspruch 4, wobei die Menge des CD66c-Rezeptor-Proteins durch einen immunologischen Test gemessen wird, umfassend das Inkontaktbringen der biologischen Probe mit einem eventuell markierten Anti-CD66c-Antikörper, der CD66c spezifisch erkennt, und den Nachweis der gebildeten CD66c-Antikörper-Rezeptor-Komplexe.

6. Verfahren nach Anspruch 5, wobei der Anti-CD66c-Antikörper ein monoklonaler Antikörper ist.

7. Verfahren nach Anspruch 6, wobei die biologische Probe eine Ileumbiopsie ist und der immunologische Test ein Immunhistochemie-Test ist.

8. Verfahren nach Anspruch 6, wobei die biologische Probe eine Blut- oder Stuhlprobe ist und der immunologische Test ein ELISA-Test ist.

9. Verfahren nach Anspruch 2, wobei das Expressionsniveau des CD66c-Rezeptors auf indirekte Weise durch die quantitative Bestimmung der Mannosemotive ermittelt wird, die im Bereich des Bürstensaums der Enterozyten vorhanden sind.

10. Verfahren nach Anspruch 9, wobei die quantitative Bestimmung der Mannosemotive wie folgt durchgeführt wird: durch das Inkontaktbringen der biologischen Probe mit Concanavalin A, das auf nachweisbare Weise markiert wurde, und den Nachweis der Komplexe, die durch das an die Mannosemotive gebundene Concanavalin A gebildet wurden.

11. Verfahren nach einem der Ansprüche 1 bis 3, wobei das Expressionsniveau des CD66c-Rezeptors durch die Messung der Menge der m-RNA von CD66c in der biologischen Probe bestimmt wird.

12. Verfahren nach Anspruch 11, wobei die biologische Probe eine Ileumbiopsie ist und Menge der m-RNA von CD66c mittels RT-PCR in Echtzeit gemessen wird.

13. Verfahren nach Anspruch 3, wobei das Expressionsniveau des CD66c-Rezeptors durch die Messung des Ausmaßes der Produktion von Anti-CD66c-Antikörpern bestimmt wird.

14. Verfahren nach Anspruch 13, umfassend das Inkontaktbringen einer zu testenden Blut- oder Stuhlprobe mit einem eventuell fixierten CD66c-Antigen oder einem Epitopfragment desselben und den Nachweis der gebildeten CD66c-Antikörper-Rezeptor-Komplexe.

15. Verwendung eines Anti-CD66c-Antikörpers, der den CD66c-Rezeptor spezifisch erkennt, zur Herstellung eines Medikaments, das zur Vorbeugung oder Behandlung von Morbus Crohn bestimmt ist.

16. Verwendung eines CD66c-Rezeptor-Proteins oder eines Glycoproteins, das CD66c nachahmt, zur Herstellung eines Medikaments, das dazu bestimmt ist, die Wechselwirkung zwischen dem CD66c-Rezeptor und den *Escherichia* Coli-Stämmen AIEC spezifisch zu blockieren, um Morbus Crohn vorzubeugen oder zu behandeln.

17. Verwendung eines Mannosids oder eines Partikels, das ein oder mehrere Mannosemotive trägt, zur Herstellung eines Medikaments, das dazu bestimmt ist, die Wechselwirkung zwischen dem CD66c-Rezeptor und den *Escherichia* Coli-Stämmen AIEC spezifisch zu blockieren, um Morbus Crohn vorzubeugen oder zu behandeln.

18. *In vitro*-Verfahren zur Auslese von Kandidatensubstanzen zur Vorbeugung oder Behandlung von Morbus Crohn, umfassend:
(i) das Inkontaktbringen des CD66c-Rezeptors in löslicher oder an der Oberfläche einer Zelle exprimierter Form mit mindestens einem *Escherichia* coli-Stamm AIEC in Gegenwart oder Abwesenheit einer zu testenden Substanz;
(ii) die Bestimmung der Fähigkeit der Substanz, die Wechselwirkung zwischen dem CD66c-Rezeptor und dem Stamm spezifisch zu hemmen, und die Selektion und/oder Identifikation der Substanz.

19. Anti-CD66c-Antikörper, der den CD66c-Rezeptor spezifisch erkennt, zur Verwendung als Medikament zur Vorbeugung oder Behandlung von Morbus Crohn.

20. Medikament, umfassend einen Anti-CD66c-Antikörper, der den CD66c-Rezeptor spezifisch erkennt, zur Vorbeugung oder Behandlung von Morbus Crohn.

21. CD66c-Rezeptor-Protein oder Glycoprotein, das CD66c nachahmt, zur Verwendung als Medikament, das dazu bestimmt ist, die Wechselwirkung zwischen dem CD66c-Rezeptor und den *Escherichia Coli*-Stämmen AIEC spezifisch zu blockieren, um Morbus Crohn vorzubeugen oder zu behandeln.

22. Medikament, umfassend ein CD66c-Rezeptor-Protein oder ein Glycoprotein, das CD66c nachahmt, das dazu bestimmt ist, die Wechselwirkung zwischen dem CD66c-Rezeptor und den *Escherichia Coli-*Stämmen AIEC spezifisch zu blockieren, um Morbus Crohn vorzubeugen oder zu behandeln.

23. Mannosid oder Partikel, das ein oder mehrere Mannosemotive trägt, zur Verwendung als Medikament, das dazu bestimmt ist, die Wechselwirkung zwischen dem CD66c-Rezeptor und den *Escherichia Coli*-Stämmen AIEC spezifisch zu blockieren, um Morbus Crohn vorzubeugen oder zu behandeln.

24. Medikament, umfassend ein Mannosid oder ein Partikel, das ein oder mehrere Mannosemotive trägt, das dazu bestimmt ist, die Wechselwirkung zwischen dem CD66c-Rezeptor und den *Escherichia Coli* -Stämmen AIEC spezifisch zu blockieren, um Morbus Crohn vorzubeugen oder zu behandeln.


### LISTE DE SEQUENCES

<110> Université d'Auvergne
<120> Procédé de diagnostic de la maladie de crohn
<130> BFF04a0022
<140>
   <141>
<160> 2
<170> PatentIn Ver. 2.1
<210> 1
   <211> 20
   <212> ADN
   <213> Séquence artificielle
<220>
   <223> Description de la séquence artificielle: amorce PCR
<400> 1
   cctgcagatt gcatgtcccc 20
<210> 2
   <211> 21
   <212> ADN
   <213> Séquence artificielle
<220>
   <223> Description de la séquence artificielle: amorce PCR
<400> 2
   ccaatacgat tctggggcag g 21